# EUROPÄISCHE PATENTSCHRIFT

(11) **EP 0 634 145 B1**
(45) Veröffentlichungstag und Bekanntmachung des Hinweises auf die Patenterteilung: **14.05.1997**
(21) Anmeldenummer: 94110566.0
(22) Anmeldetag: 07.07.1994
(51) Int. Cl.: A61B 17/16

(54) **Hohlschaftraspel**
Rasp with hollow shaft
Râpe avec tige creuse

(30) Priorität: 16.07.1993 DE 4323873
(43) Veröffentlichungstag der Anmeldung: 18.01.1995
(73) Patentinhaber: ESKA Implants GmbH & Co., 23556 Lübeck (DE)
(72) Erfinder: Ritter, Gebhard, Prof. Dr., D-55126 Mainz (DE); Grundei, Hans, Dr., D-23558 Lübeck (DE)
(74) Vertreter: Fuchs, Luderschmidt & Partner Patentanwälte

(56) Entgegenhaltungen:
- EP-A- 0 331 626
- DE-A- 3 433 105
- DE-U- 9 212 906
- US-A- 4 819 635
- US-A- 5 006 121
- US-A- 5 203 653

## Beschreibung

Die Erfindung betrifft eine Hohlschaftraspel.

Vor der Implantation einer stielförmigen Endoprothese in einen Röhrenknochen, beispielsweise in den menschlichen Femur, wird dieser an seinem gelenkseitigen Ende reseziert und eine Ausfräsung der Spongiosa und Entfernung von Knochenmark vorgenommen. In den ausgefrästen Raum kann sodann der Stiel der Endoprothese eingebracht werden, wo er zementlos oder unter Anwendung von Zement (Methylmetacrylat) fixiert wird. Ein typisches Beispiel für eine derartige stielförmige Endoprothese ist der Hüftstiel eines künstlichen Hüftgelenks.

Häufig verwendete und aus der Praxis bekannte Werkzeuge zum Ausräumen des Spongiosaraumes bestehen aus einer massiven Raspel, welche die Form des Stielteils der Endoprothese aufweist und an deren proximalen Ende ein Handgriff für die Handhabung durch den Operateur angebracht ist.

Bereits dieser Operationsschritt des Ausräumens des Spongiosa und Markraumes ist mit erheblichen Risiken verbunden. So ist beobachtet worden (Wenda et. al. in "Unfallchirurg", 1990, Seiten 56 bis 61), daß bereits beim Aufraspeln des Spongiosaraumes vermehrt Embolien und/oder Thrombosen auftreten, die in der Praxis während der Operation zu zusätzlichen schwersten Komplikationen aber auch zum Tode des Patienten führen können. Ein Grund hierfür ist die vermehrte Knochenmarksfettausschüttung, welche zu Fettembolien beim Patienten führen können.

Eine Knochenraspel mit einem im Inneren vorgesehenen Kanal ist bekannt aus der DE-39 07 256 A1, mit der versucht wird, beim Ausfräsen eines Röhrenknochens abgetrennte Knochenspäne und Knochenmarksstückchen in das Innere der Raspel zu leiten und von dort gegebenenfalls mit einer speziellen Vorrichtung abzusaugen. Diese Hohlschaftraspel besteht in einer konkreten Ausführungsform aus dem Raspelkörper, in welchem der Sammelkanal für die Knochenspäne und Knochenmarksstückchen ausgebildet ist als eine Sackbohrung. Die Späne etc. sollen durch in der Wandung vorgesehene Durchtrittsöffnungen von außen nach innen in die Raspel eintreten.

Die Knochenraspel gemäß der genannten Druckschrift schneidet oder spant sowohl beim Hineinstoßen der Raspel in den Knochenmarksraum, als auch beim Herausziehen. Nun ist die Konsistenz der im Markraum befindlichen Masse so, daß die erwähnten Durchtrittsöffnungen sehr schnell verstopfen, so daß die Raspel dann lediglich die Wirkung einer weiter oben erwähnten massiven Raspel hat.

Vor diesem Hintergrund ist es nun die Aufgabe der vorliegenden Erfindung, ein Werkzeug anzugeben, mit dem der Spongiosa- und Markraum eines Röhrenknochens zur Präparation einer Implantation ausgeräumt werden kann, bei dessen Anwendung das Risiko von Thrombosen und/oder Embolien erheblich reduziert ist, wobei die aufgeführten Nachteile der vorerwähnten Knochenraspel mit Innenkanal vermieden werden sollen.

Erfindungsgemäß besteht das Werkzeug aus einer Hohlschaftraspel, an welche proximal ein Handgriff für die Handhabung durch den Operateur anbringbar ist. Die Hohlschaftraspel besteht aus einem Hohlrohr, in dessen Wandung ein Schabeprofil eingearbeitet ist. Dieses Schabeprofil wirkt nur unidirektional, vorzugsweise beim Eindringen in den Röhrenknochen. Wird die Raspel aus dem Röhrenknochen herausgezogen, wirkt das Schabeprofil nicht. Im Gegensatz hierzu wirkt die Raspel gemäß der DE-39 07 256 A1 in beiden Richtungen, was neben der relativ hohen Wandungsdicke zu einem schnellen Verstopfen der Durchtrittsöffnungen führt.

Die Wirkung der erfindungsgemäßen Hohlschaftraspel ist die folgende: beim Vorantreiben der Raspel in den Röhrenknochen wird Spongiosa und Knochenmark in das Innere des Hohlrohres gedrängt. Im Gegensatz dazu wurde bei herkömmlichen Werkzeugen das Knochenmark und die Spongiosa gegen die Knocheninrienwand gedrückt mit der Folge, daß zumindest Knochenmarkfett in die Blutbahn des Patienten übertreten konnte und so zu Fettembolien führte. Vorliegend nun sammelt sich sämtlicher Abraum im Inneren der Hohlschaftraspel.

Erfindungsgemäß ist die Hohlschaftraspel medial mit einem Längsschlitz versehen. Dieser Längsschlitz ist vorteilhaft auf der ganzen Länge von distal nach proximal vorgesehen. Er verleiht der Hohlschaftraspel eine federnde Wirkung in ihrem Umfangsbereich. Hierdurch kann die Wandung der Raspel eine Ausgleichsbewegung in Abhängigkeit der jeweilig herrschenden Druckverhältnisse ausführen.

Gemäß einer besonders vorteilhaften Ausführungsform weist die erfindungsgemäße Raspel proximal ein Anschlußstück auf, an das eine Absaugeinheit anschließbar ist. Dies ist für sich genommen bereits aus der obengenannten Druckschrift DE-39 07 256 A1 bekannt. Hierdurch wird das Risiko einer Thrombose oder Embolie noch weiter reduziert, da es nämlich möglich ist, durch das Aufraspeln bereits in das Innere des Hohlrohres gelangtes Spongiosa- und Knochenmarksmaterial nach außen abzusaugen, so daß es in keinem Falle zu einem Stau vom Material im Hohlrohrinneren kommen kann.

Vorteilhaft weist die erfindungsgemäße Hohlschaftraspel im wesentlichen die Außenkontur des Stieles der zu implantierenden Endoprothese auf, wie schon aus der Zeichnungsfigur der genannten Druckschrift bekannt. Gemäß dieser Ausführungsform wird die Hohlschaftraspel also nicht die Form eines hohlzylindrischen Rohres aufweisen. Der Vorteil dieser Formgebung besteht darin, daß der auszufräsende Raum von vorn herein die Form annehmen wird, damit der Stiel der Endoprothese genau dort hineinpaßt.

An dieser Stelle sei noch auf die DE-92 17 486 U1 hingewiesen, die ebenfalls eine Raspel zur Vorbereitung einer Knochenöffnung betrifft. Aus dieser ist allerdings keine Hohlschaftraspel bekannt, sondern das Prinzip des lösbaren Griffes am proximalen Ende der Raspel, wie dies im Oberbegriff des Anspruchs 1 vorausgesetzt wird.

Die Erfindung wird anhand eines Ausführungsbeispieles gemäß der Zeichnungsfiguren näher erläutert. Hierbei zeigt:
- Fig. 1: eine Seitenansicht eines Ausführungsbeispieles der Hohlschaftraspel, teilweise geschnitten, und
- Fig. 2: die Ansicht auf die Raspel gemäß Fig. 1 von medial gesehen.

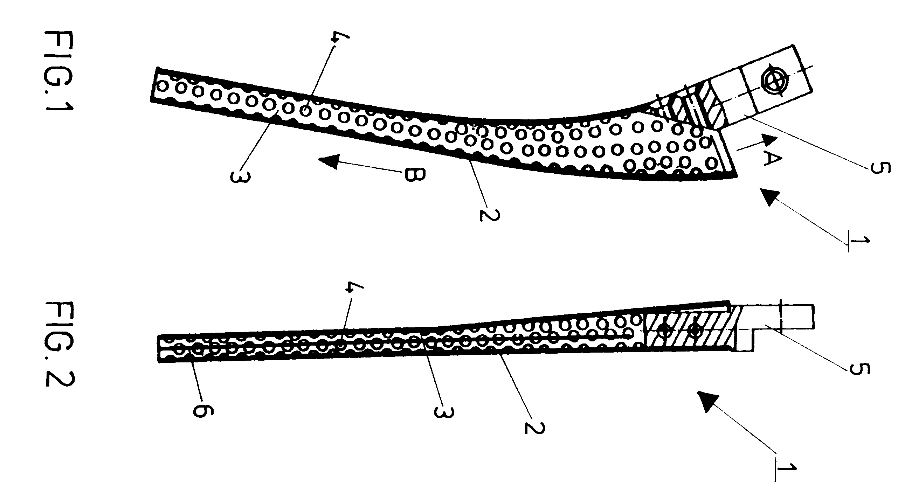

In den Zeichnungsfiguren ist eine Hohlschaftraspel 1 dargestellt, die für das Ausräumen des menschlichen Femurknochens im Bereich des Hüftgelenks ausgebildet ist. Daher erinnert die Form (Fig. 1) auch an die Form eines Hüftstiels eines künstlichen Hüftgelenks.

Proximal weist die Hohlschaftraspel ein Anschlußstück 5 für einen Handgriff auf. Dieses ist in geeigneter Form ausgebildet und mit einem Teil in den proximalen Teil der Raspel eingelassen und dort befestigt. Die Hohlschaftraspel 1 besteht aus einem Hohlrohr 2. Dessen Wandung 3 ist mit einem Schabeprofil 4 versehen, das beispielsweise durch Stanzen eines Bleches vor dem Aufrollen um einen Dorn hergestellt sein kann. Das Schabeprofil 3 ist so ausgebildet, daß es nur in einer Bewegungsrichtung der Raspel wirkt, vorzugsweise bei Bewegung der Raspel 1 in Richtung des Pfeiles B in Fig. 1. Dabei wird das Abraummaterial in das Innere des Hohlrohres 2 gedrängt.

Gemäß einer besonders bevorzugten Ausführung kann vorgesehen sein, am proximalen Abschnitt der Raspel 1 eine Absaugeinheit (nicht dargestellt) anzuschließen, durch welche das Abraummaterial abgesogen werden kann. Dies soll der Pfeil A in Fig. 1 versinnbildlichen.

Aus Fig. 2 ist ersichtlich, daß von medial gesehen ein Längsschlitz von distal nach proximal in der Wandung 3 des Hohlrohres 2 vorgesehen ist. Der Schlitz verleiht der Raspel eine gewisse ausgleichende Federwirkung im Umfangsbereich.

## Patentansprüche

1. Hohlschaftraspel (1) zur Präparation eines Röhrenknochens für den Einsatz einer stielförmigen Endoprothese, an welche proximal ein Handgriff anbringbar ist und welche aus einem Hohlrohr (2) besteht, in dessen Wandung (3) ein unidirektional wirkendes Schabeprofil (4) eingearbeitet ist, dadurch gekennzeichnet, daß das Hohlrohr (2) medial einen Längsschlitz (6) aufweist, der dem Hohlrohr (2) eine federnde Wirkung verleiht.

2. Hohlschaftraspel nach Anspruch 1, bei der proximal ein Anschlußstück vorgesehen ist, an das eine Absaugeinheit anschließbar ist.

3. Hohlschaftraspel nach Anspruch 1 oder 2, die im wesentlichen die Außenkontur des Stieles der zu implantierenden Endoprothese aufweist.

## Claims

1. Hollow shaft rasp (1) for preparation of a tubular bone for using a stem-shaped endoprosthesis, to which a hand grip can be attached proximally and which comprises a hollow tube (2), into the wall (3) of which is incorporated a unidirectionally acting scraping profile (4), characterised in that the hollow tube (2) has a longitudinal slot (6) medially, which imparts a spring effect to the hollow tube (2).

2. Hollow shaft rasp according to claim 1, in which a connection piece, to which a suction unit can be connected, is provided proximally.

3. Hollow shaft rasp according to claim 1 or 2, which essentially has the external contour of the stem of the endoprosthesis to be implanted.

## Revendications

1. Râpe tubulaire creuse (1) pour la préparation d'un os tubulaire pour la mise en place d'une endoprothèse en forme de tige, sur le côté proximal de laquelle peut être montée une poignée et qui est constituée par un tube creux (2), dans la paroi (3) duquel est aménagé un profil de racle (4) agissant de façon unidirectionnelle, caractérisée en ce que le tube (2) comporte, sur le côté médial, une fente longitudinale (6), qui confère au tube creux (2) une action élastique.

2. Râpe tubulaire creuse selon la revendication 1, dans laquelle est prévu, côté proximal, un élément de raccordement auquel peut être raccordée une unité d'aspiration.

3. Râpe tubulaire creuse selon la revendication 1 ou 2, qui possède essentiellement le profil extérieur de la tige de l'endoprothèse devant être implantée.
